# EUROPÄISCHE PATENTSCHRIFT

(11) **EP 2 794 166 B1**
(45) Veröffentlichungstag und Bekanntmachung des Hinweises auf die Patenterteilung: **05.04.2017**
(21) Anmeldenummer: 12784566.7
(22) Anmeldetag: 07.11.2012
(51) Int. Cl.: B23D 61/02

(54) **DREHOSZILLATIONSSÄGEBLATT FÜR EINE WERKZEUGMASCHINE**
ROTARY RECIPROCATING SAW BLADE FOR A POWER TOOL
LAME DE SCIE OSCILLANTE ROTATIVE POUR UNE MACHINE-OUTIL

(30) Priorität: 20.12.2011 DE 102011089097
(43) Veröffentlichungstag der Anmeldung: 29.10.2014
(73) Patentinhaber: Robert Bosch GmbH, 70442 Stuttgart (DE)
(72) Erfinder: BOZIC, Milan, CH-4500 Solothurn (CH)
(86) Internationale Anmeldenummer: PCT/EP2012/071980
(87) Internationale Veröffentlichungsnummer: WO 2013/091979

(56) Entgegenhaltungen:
- EP-A1- 0 695 607
- WO-A1-01/66023
- DE-A1- 10 231 393

## Beschreibung

Die Erfindung bezieht sich auf ein Drehoszillationssägeblatt für eine Werkzeugmaschine nach dem Oberbegriff des Anspruches 1.

### Stand der Technik

In der EP 1 263 333 B1 wird ein Tauchsägeblatt für eine Werkzeugmaschine beschrieben, das im Betrieb eine oszillierende Drehbewegung ausführt. Das Tauchsägeblatt weist ein annähernd rechteckförmiges Stammblatt auf, das einenends mit einem Halterungsabschnitt zur Verbindung mit der Werkzeugwelle und anderenends mit einer Schneidkante mit Schneidzähnen versehen ist. In die Seitenfläche des Stammblattes sind zwischen der Aufnahme für die Werkzeugwelle und der Schneidkante eine Vielzahl kleinflächiger Einprägungen eingebracht, welche dazu beitragen sollen, das Aufschwenkverhalten des Sägeblattes im Betrieb zu reduzieren und die Schnittpräzision zu erhöhen. Die Einprägungen weisen eine verhältnismäßig geringe Einprägetiefe auf und können als Rechtecke, Kreise oder Dreiecke ausgebildet sein, wobei die nicht-runden Geometrien eine zur Längsachse des Tauchsägeblatts winklige Ausrichtung besitzen. Insgesamt sind in die Seitenfläche eine Vielzahl von Einprägungen eingebracht.

### Offenbarung der Erfindung

Der Erfindung liegt die Aufgabe zugrunde, mit einfachen konstruktiven Maßnahmen ein Drehoszillationssägeblatt für eine Tauchsäge so auszubilden, dass über einen langen Betriebszeitraum eine präzise Werkstückbearbeitung möglich ist.

Diese Aufgabe wird erfindungsgemäß mit den Merkmalen des Anspruches 1 gelöst. Die Unteransprüche geben zweckmäßige Weiterbildungen an.

Die Erfindung bezieht sich auf ein Drehoszillationssägeblatt in Form eines Tauchsägeblattes, das in Werkzeugmaschinen, insbesondere in handgeführten Werkzeugmaschinen eingesetzt werden kann und im Betrieb eine Drehoszillationsbewegung ausführt. Das Tauchsägeblatt weist ein beispielsweise kreissegmentförmiges oder rechteckförmiges bzw. zumindest annähernd rechteckförmiges Stammblatt auf, dessen eine Seitenkante als zumindest annähernd geradlinige, gegebenenfalls leicht gebogene Schneidkante mit Schneidzähnen ausgebildet ist.

In mindestens eine Seitenfläche des Stammblattes ist mindestens eine Einprägung eingebracht, die vorzugsweise linien- oder streifenförmig ausgebildet ist und sich beispielsweise in Richtung der Schneidkante erstreckt. Die Einprägung verändert die Spannungseigenschaften des Stammblattes, wodurch sowohl die Eigenfrequenz des Tauchsägeblattes als auch die Ausbreitung von Vibrationswellen positiv beeinflusst werden. Es kann eine Überlappung der Eigenfrequenzen von Stammblatt und Werkzeugmaschine verhindert werden, was zu einer verringerten Schwingung des Stammblattes im Bereich der Schneidkante mit den Schneidzähnen führt. Insgesamt wird die Vibrations- und die Geräuschentwicklung reduziert, so dass neben einer Verbesserung des Komforts auch die Bearbeitungsqualität gesteigert ist. Aufgrund der geringeren Belastungen, denen das Tauchsägeblatt unterliegt, kann die verbesserte Arbeitsqualität auch über einen längeren Betriebszeitraum aufrechterhalten werden.

In das Stammblatt ist zumindest eine Aussparung eingebracht, die von mindestens einer Einprägung begrenzt ist. Die Aussparung, welche beispielsweise durch Stanzen erzeugt werden kann, reduziert das Massenträgheitsmoment des Stammblattes, was einen positiven Effekt, insbesondere im Hinblick auf eine geringere Schwingungsübertragung, auf die Standzeit und die Bedienerfreundlichkeit hat. Außerdem können kleiner dimensionierte Antriebsmotoren eingesetzt werden. Die Einprägung im Stammblatt wirkt einer durch die Ausnehmung hervorgerufenen Stabilitätsminderung entgegen. Somit können durch die Kombination von Aussparung und Einprägung verschiedene Vorteile verbunden werden, nämlich eine verringerte Masse bzw. ein Massenträgheitsmoment und eine verbesserte Stabilität mit verringerter Schwingungsausbreitung.

Vorteilhafterweise sind in das Stammblatt mehrere Aussparungen eingebracht, die jeweils von zumindest einer, vorzugsweise linien- bzw. streifenförmigen Einprägung begrenzt sind. Es kann zweckmäßig sein, an mindestens zwei Seiten einer Aussparung, gegebenenfalls an allen Seiten der Aussparung Einprägungen vorzusehen, um der Verringerung der Stabilität durch die Aussparungen entgegenzuwirken.

Es kommen verschiedene Querschnittsgeometrien der Aussparungen in Betracht, insbesondere eckige, beispielsweise dreieckige oder rechteckige Geometrien.

Vorteilhaft ist des Weiteren, dass die Einprägung in einer einfachen Weise hergestellt werden kann. Die Einprägung führt zu einer Verfestigung im Stammblatt und damit einhergehend zu einer veränderten Spannungseigenschaft.

Die Einprägung ist durch Prägen erzeugt. Auf einer Seitenfläche des Stammblattes ist eine Einprägung in Form einer Vertiefung vorhanden, auf der gegenüberliegenden Seitenfläche erzeugt das Prägen dagegen eine korrespondierende Erhöhung. Die durch Prägen erzeugten Einprägungen weisen eine Einprägtiefe von vorzugsweise mindestens 0.1 mm auf. Die Einprägungen können alle in die gleiche Seitenfläche eingebracht sein, so dass an dieser Seitenfläche nur Vertiefungen und an der gegenüberliegenden Seitenfläche nur Erhebungen vorhanden sind. Alternativ ist es auch möglich, an jeder Seitenfläche Einprägungen vorzusehen, so dass jede der beiden Seitenflächen Vertiefungen und Erhöhungen aufweist.

Es kommen verschiedenartig ausgeführte Einprägungen in Betracht. Möglich sind linienförmige oder streifenförmige Einprägungen, welche entweder geradlinig oder gekrümmt ausgeführt sind bzw. geradlinige und gekrümmte Abschnitte aufweisen können. Im Falle einer linienförmigen Einprägung ist die Breite sehr gering, im Falle einer streifenförmigen Einprägung liegt die Breite vorzugsweise bei maximal ein Drittel der Blatthöhe, beispielsweise 3 mm, wobei ggf. auch größere Breiten möglich sind. Grundsätzlich möglich sind auch flächige Einprägungen auf dem Sägeblatt.

Des Weiteren ist es möglich, insbesondere im Falle einer linienförmigen Einprägung, umlaufend geschlossen ausgebildete geometrische Muster zu erzeugen, beispielsweise annähernd rechteckförmige Einprägungen, dreieckförmige Einprägungen oder dergleichen. Die Einprägung an sich ist linien- oder streifenförmig und bildet die Randkontur des geometrischen Musters.

Es kann zweckmäßig sein, dass sich die Einprägung im Falle einer linien- oder streifenförmigen Ausbildung zumindest abschnittsweise in Längsrichtung, also parallel zur Schneidkante erstreckt. Grundsätzlich möglich ist aber auch eine winklige Ausrichtung der linien- bzw. streifenförmigen Einprägung gegenüber der Längsachse oder ein orthogonaler Verlauf, also in Querrichtung des Sägeblattes. Des Weiteren sind verschiedenartige Muster von Einprägungen möglich, welche als Kombination unterschiedlicher Einprägungen darstellbar sind, beispielsweise eine Kombination geradliniger Einprägungen, die abschnittsweise in Längs- und in Querrichtung bzw. unter einem Winkel zur Längsachse verlaufen.

Möglich sind des Weiteren definierte Muster von Einprägungen, die sich in Abhängigkeit der axialen Position ändern.

Die linien- bzw. streifenförmigen Einprägungen sind so in das Stammblatt eingebracht, dass zumindest zwei Eckpunkte des Stammblattes von den Einprägungen verbunden werden. Vorzugsweise werden alle vier Eckpunkte des Stammplatzes von linien- bzw. streifenförmigen Einprägungen verbunden, wobei grundsätzlich verschiedene Ausführungsvarianten in Betracht kommen. Möglich ist es beispielsweise, diagonal verlaufende Einprägungen einzubringen, die diagonal liegende Eckpunkte des Stammblattes verbinden. In Betracht kommt zusätzlich oder alternativ eine Verbindung über in Längsrichtung bzw. in Querrichtung verlaufende Einprägungen zwischen den Eckpunkten im Stammblatt. Sowohl mit diagonalen als auch mit sich in Längsrichtung bzw. quer zur Längsrichtung erstreckenden Einprägungen wird eine Rahmenkonstruktion mit Einprägungen geschaffen, welche die Festigkeit des Stammblattes in signifikanter Weise verbessert. Die diagonalen und/oder sich in Längsrichtung bzw. Querrichtung erstreckenden Einprägungen können bis zu den jeweiligen Eckpunkten verlaufen. Gemäß weiterer Ausführung ist es aber auch möglich, dass eine derartige Einprägung auf Abstand zu einem oder zwei Eckpunkten liegt, wobei in diesem Fall die Längsachse der Einprägung durch die Eckpunkte führt.

Gegebenenfalls können lokal im Sägeblatt zusätzlich zu den Einprägungen chemische Nachbearbeitungen, beispielsweise durch Beschichten vorgenommen werden, um die Spannung im Sägeblatt zu beeinflussen. Alternativ oder zusätzlich sind auch lokal thermische Nachbearbeitungen, beispielsweise Induktionshärten, möglich.

Weitere Vorteile und zweckmäßige Ausführungen sind den weiteren Ansprüchen, der Figurenbeschreibung und den Zeichnungen zu entnehmen. Es zeigen:
- Fig. 1: ein als Tauchsägeblatt ausgeführtes Drehoszillationssägeblatt mit einem annähernd rechteckförmigen Stammblatt, dessen Vorderkante als Schneidkante mit Schneidzähnen ausgeführt ist, und mit einem Halterungsteil zur Befestigung an einer Werkzeugwelle, sowie mit mehreren Einprägungen im Stammblatt, welche zwischenliegende Aussparungen begrenzen,
- Fig. 2: das Stammblatt aus Fig. 1 in einem Schnitt gemäß Schnittlinie II-II,
- Fig. 3: ein Tauchsägeblatt in einer weiteren Ausführung,
- Fig. 4: das Tauchsägeblatt in einer weiteren Ausführung,
- Fig. 5: ein Tauchsägeblatt, dessen Stammblatt seitliche Einbuchtungen aufweist sowie mit Einprägungen und Aussparungen versehen ist,
- Fig. 6: ein ähnliches Tauchsägeblatt wie Fig. 5, jedoch mit unterschiedlich ausgeführten Einprägungen,
- Fig. 7: noch ein weiteres Tauchsägeblatt gemäß Fig. 5 oder 6 mit Einprägungen in noch einer weiteren Ausführungsvariante.

In den Figuren sind gleiche Bauteile mit gleichen Bezugszeichen versehen.

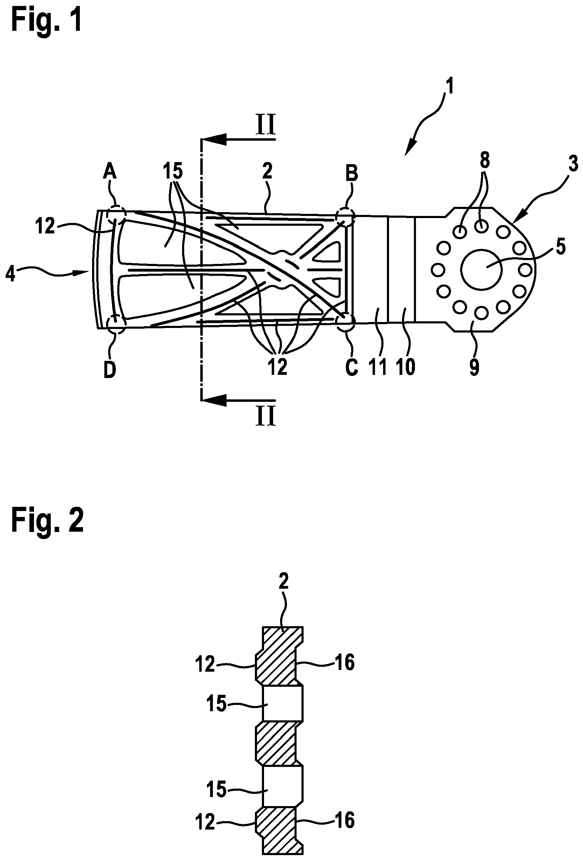

Das in Fig. 1 dargestellte Tauchsägeblatt 1 für eine Handwerkzeugmaschine mit Drehoszillationsantrieb umfasst ein zumindest annähernd rechteckförmiges oder kreissegmentförmiges Stammblatt 2 sowie ein mit dem Stammblatt 2 verbundenes, gekröpftes Halterungsteil 3. An der freien, dem Halterungsteil 3 abgewandten Stirnseite ist das Stammblatt 2 mit einer Schneidkante 4 mit Schneidzähnen versehen. Das Halterungsteil 3 umfasst einen Wellenaufnahmeabschnitt 9 mit einer Befestigungsaufnahme 5 zur Aufnahme der Werkzeugwelle 6 der Werkzeugmaschine, welche eine drehoszillierende Bewegung um die Drehachse 7 ausführt. Die Befestigung mit der Werkzeugwelle 6 erfolgt mittels Rastöffnungen 8, die ringförmig um die zentrale Befestigungsausnehmung 5 in dem Wellenaufnahmeabschnitt 9 eingebracht sind.

Das Halterungsteil 3 umfasst des Weiteren einen Übergangsabschnitt 10 sowie einen Verbindungsabschnitt 11, über den die Verbindung zu dem Stammblatt 2 erfolgt; Stammblatt 2 und Halterungsteil 3 sind als separate Bauteile ausgeführt, die jedoch fest miteinander verbunden sind. Der Wellenaufnahmeabschnitt 9 und der Verbindungsabschnitt 11 liegen parallel versetzt zueinander, die Höhendifferenz wird von dem schräg verlaufenden Übergangsabschnitt 10 überbrückt. Der Wellenaufnahmeabschnitt 9, der Übergangsabschnitt 10 und der Verbindungsabschnitt 1 sind einteilig ausgebildet.

Wie Fig. 1 des Weiteren zu entnehmen, weist das Stammblatt 2 des Tauchsägeblattes 1 eine Mehrzahl von Aussparungen 15 auf, welche das Stammblatt orthogonal zur Stammblattebene vollständig durchsetzen. Grundsätzlich möglich sind aber auch Aussparungen in Form von Vertiefungen im Stammblatt 2, die das Stammblatt nicht vollständig durchsetzen; in diesem Fall können die Vertiefungen entweder nur an einer Seitenfläche des Stammblattes 2 oder an beiden Seitenflächen angeordnet sein, jeweils in spiegelsymmetrischer oder in asymmetrischer Weise.

Durch das Einbringen der Aussparungen 15 werden die Masse und das Massenträgheitsmoment des Stammblattes sowie des Tauchsägeblattes reduziert. Um die mit den Aussparungen verbundene Steifigkeitsschwächung zumindest teilweise zu kompensieren, sind in das Stammblatt Einprägungen 12 eingebracht, die vorzugsweise nur auf einer Seitenfläche des Stammblattes angeordnet sind. Die Einprägungen 12 sind insbesondere in der Weise auf die Seitenfläche aufgebracht, dass die Aussparungen 15 an mehreren, vorzugsweise an allen Seiten von den Einprägungen begrenzt werden. Die Einprägungen bewirken eine Festigkeitssteigerung.

Die Einprägungen werden bevorzugt durch Prägen erzeugt; in diesem Fall sind nur auf einer Seitenfläche des Stammblattes Einprägungen in Form einer Vertiefung von mindestens 0.1 mm vorhanden, auf der gegenüberliegenden Seitenfläche erzeugt das Prägen dagegen eine korrespondierende Erhöhung. Mit dem Erzeugen der Einprägungen 12 wird kein Material vom Stammblatt abgetragen.

Es sind insgesamt eine Vielzahl von Einprägungen 12 in das Stammblatt 2 des Tauchsägeblattes 1 eingebracht, die sich in Längsrichtung - orthogonal zur Schneidkante 4 -, in Querrichtung bzw. winklig zur Längsachse, beispielsweise diagonal erstrecken. Mehrere Einprägungen 12 verlaufen zwischen den Eckpunkten A, B, C und D des annähernd rechteckförmigen Stammblattes 2. Die Einprägungen 12 sind geradlinig bzw. - im Fall diagonal verlaufender Einprägungen - gekrümmt ausgeführt. Die virtuellen Eckpunkte des Stammblattes 2 werden von den Einprägungen 12 verbunden, und zwar sowohl in Diagonalrichtung als auch in Längsrichtung bzw. quer zur Längsachse des Stammblattes. Die Verbindung erfolgt in der Weise, dass sich Einprägungen bis zu den Eckpunkten A, B, C, D bzw. mit nur verhältnismäßig geringem Abstand zu den Eckpunkten erstrecken. Jeder Eckpunkt wird im Ausführungsbeispiel gemäß Fig. 1 von einer gekrümmt verlaufenden diagonalen Einprägung, von einer sich in Längsrichtung erstreckenden Einprägung sowie einer sich in Querrichtung erstreckenden Einprägung geschnitten.

Die Einprägungen 12 verlaufen entlang von Stegen im Stammblatt 2. Zur Stabilisierung des Stammblattes ist ein mittlerer, sich in Längsrichtung erstreckender Steg mit einer Einprägung 12 vorgesehen. Insgesamt sind zwischen den Einprägungen 12 im Ausführungsbeispiel sechs Aussparungen 15 unterschiedlicher Größe, jeweils mit zumindest annähernd dreieckförmiger Geometrie eingebracht. Sämtliche Aussparungen 15 sind an allen Seiten von Stegen bzw. Einprägungen 12 begrenzt.

Wie der Schnittdarstellung gemäß Fig. 2 zu entnehmen, bewirkt jede durch Prägen erzeugte Einprägung 12 in Form einer Vertiefung an einer Seitenfläche des Stammblattes 2 eine korrespondierende Erhöhung 16 an der gegenüberliegenden Seitenfläche des Stammblattes. Die Vertiefungen bzw. Erhöhungen begrenzen die zwischenliegenden Aussparungen 15.

Sich kreuzende Einprägungen liegen nicht übereinander, vielmehr ist in diesem Fall nur eine der Einprägungen durchgehend und die sich kreuzende Einprägung mit Unterbrechung ausgebildet.

Insgesamt bilden die Einprägungen eine rahmenförmige Konstruktion zwischen den Eckpunkten A, B, C, D, welche die Festigkeit des Stammblattes 2 in signifikanter Weise verbessert. Es kann grundsätzlich ausreichend sein, die Eckpunkte beispielsweise nur durch Diagonalen zu verbinden, oder, alternativ hierzu, nur durch sich in Längsrichtung bzw. Querrichtung erstreckende Einprägungen. Des Weiteren ist es möglich, so wie dies in den Ausführungsbeispielen gemäß den Figuren 1, 3 und 4 dargestellt ist, die Eckpunkte sowohl durch diagonal verlaufende Einprägungen als auch durch in Längsrichtung und in Querrichtung verlaufende Einprägungen zu verbinden.

Gemäß Fig. 1 erstrecken sich die diagonal verlaufenden Einprägungen jeweils zwischen diagonal gegenüberliegenden Eckpunkten A, C bzw. B, D, wohingegen die sich in Längsrichtung erstreckenden Einprägungen zumindest bei den der vorderen Schneidkante 4 benachbarten Eckpunkten A, D verkürzt ausgebildet sind und zu diesen Eckpunkten einen größeren Abstand aufweisen. Gleichwohl schneiden die Längsachsen der Einprägungen 12, welche sich in Längsrichtung erstrecken, trotz eines ggf. vorhandenen Abstandes die Eckpunkte.

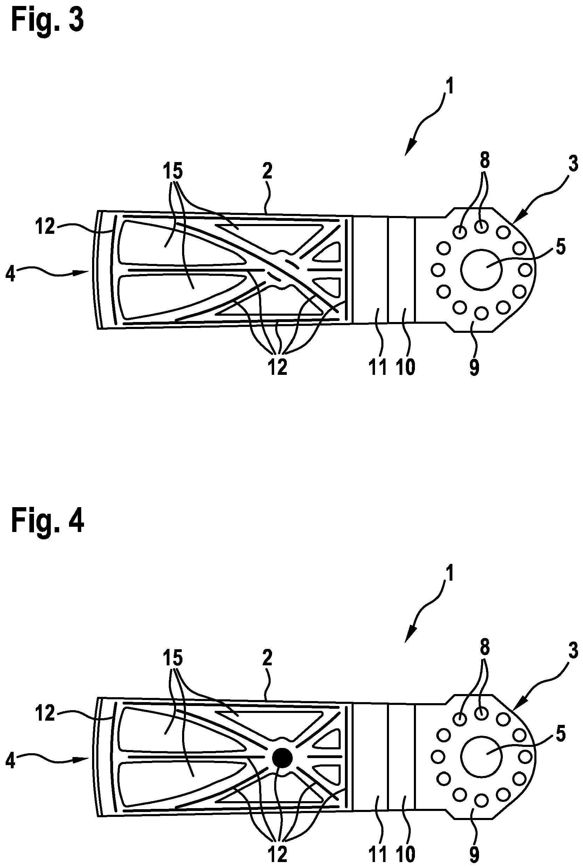

Im Ausführungsbeispiel gemäß Fig. 2 weist das Stammblatt 2 des Tauchsägeblattes 1 eine grundsätzliche ähnliche Gestalt auf wie im Ausführungsbeispiel gemäß Fig. 1. Allerdings erstrecken sich die diagonal verlaufenden Einprägungen 12 beim Ausführungsbeispiel gemäß Fig. 3 nicht bis zu den vorderen Eckpunkten, sondern weisen zu diesen einen Abstand auf. Dagegen erstrecken sich die randseitigen, in Längsrichtung verlaufenden Einprägungen bis zu den vorderen und auch den hinteren Eckpunkten.

Auch die randseitigen Einprägungen, welche in Längsrichtung verlaufen, weisen einen zumindest geringfügigen Abstand zur jeweiligen Seitenkante des Stammblattes 2 auf.

Die in Querrichtung verlaufenden Einprägungen auf der der Schneidkante 4 benachbarten Seite bzw. der dem Halterungsteil 3 benachbarten Seite erstrecken sich über die gesamte Breite des Stammblattes 2. In Fig. 1 weisen dagegen die sich in Querrichtung verlaufenden Einprägungen zu jeder Seitenkante einen Abstand auf.

In Fig. 4 ist das Stammblatt 2 im Wesentlichen gleich wie in Fig. 3 ausgebildet, jedoch mit dem Unterschied, dass die sich kreuzenden, diagonal verlaufenden Einprägungen im Kreuzungspunkt beide unterbrochen sind, wobei im Bereich des Kreuzungspunktes eine kleine kreisförmige, flächige Einprägung eingebracht ist.

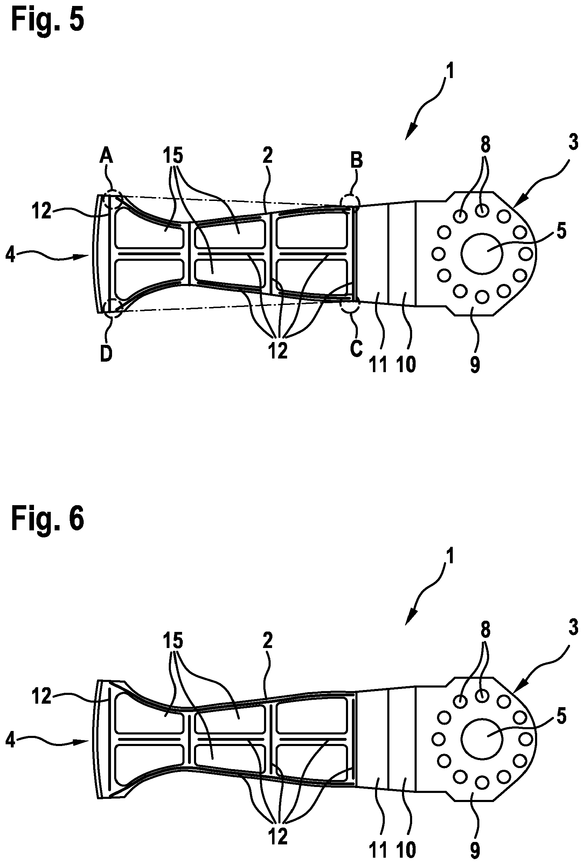

In den Figuren 5 bis 7 sind weitere Ausführungsbeispiele für Tauchsägeblätter 1 dargestellt. Die Stammblätter 2 der Tauchsägeblätter weisen gegenüber der Verbindungslinie zwischen vorderem und hinterem Eckpunkt A, B bzw. C, D eine seitliche Einbuchtung auf, wodurch das Gewicht und das Trägheitsmoment des Tauchsägeblatts 1 weiter reduziert wird. Wie bei den ersten Ausführungsbeispielen erstrecken sich auch gemäß Fig. 5 bis 7 die Einprägungen 12 zwischen den Eckpunkten A, B, C, D des Stammblattes 2. Auf Grund der nicht-geradlinigen Seitenkante des Stammblattes 2 sind auch die unmittelbar benachbart und parallel zur Seitenkante verlaufenden Einprägungen zumindest abschnittsweise gekrümmt ausgeführt. Zusätzlich gibt es in einem mittleren, sich in Längsrichtung erstreckenden Steg mehrere Einprägungsabschnitte sowie sich in Querrichtung erstreckenden Stegen Einprägungen zwischen den beiden Seitenkanten. Die vorne liegenden Seitenkanten A, D sowie die hinten liegenden Eckpunkte B, C werden jeweils von sich in Querrichtung erstreckenden Einprägungen geschnitten.

In das Stammblatt 2 sind insgesamt sechs Aussparungen 15 eingebracht, die jeweils an jeder Seite von Einprägungen 12 begrenzt sind. Die Aussparungen 15 weisen eine annähernd viereckige Form auf, wobei im Bereich der stärksten Krümmung der Seitenkante des Stammblattes 2 auch die Aussparungen eine entsprechend gekrümmte Seitenlinie besitzen.

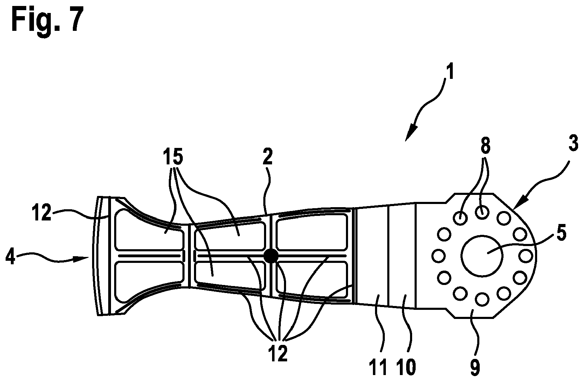

Im Ausführungsbeispiel gemäß Fig. 5 erstrecken sich die in Querrichtung verlaufenden Einprägungen 12 zwischen den vorderen Eckpunkten A, D bzw. den hinteren Eckpunkten B, C über die gesamte Breite des Stammblattes, gleiches gilt für das Ausführungsbeispiel gemäß Fig. 7, wohingegen gemäß Fig. 6 die Einprägungen 12 zwischen den vorderen bzw. hinteren Eckpunkten auf Abstand zu den Eckpunkten liegen und die entlang der Seitenkanten erstreckenden Einprägungen bis zu den Eckpunkten verlaufen.

Fig. 7 ist außerdem zu entnehmen, dass im Kreuzungsbereich von in Längsrichtung und in Querrichtung verlaufenden Einprägungen verschiedenartige Unterbrechungen vorhanden sein können. Möglich ist z.B. eine flächige, ovale oder kreisförmige Einprägung, die auf dem mittleren, sich in Längsrichtung erstrecken Steg liegt, oder ein Absetzen der sich in Längsrichtung erstreckenden Einprägung mittels zweier kurzer, an beiden Seiten der sich in Querrichtung erstreckenden Einprägung vorhandenen kleinerer Einprägungsabschnitte.

## Patentansprüche

1. Drehoszillationssägeblatt für eine Werkzeugmaschine, insbesondere für eine Handwerkzeugmaschine, mit einem Stammblatt (2), das eine zumindest annähernd geradlinige Schneidkante (4) aufweist, wobei in eine Seitenfläche des Stammblatts (2) mindestens eine Einprägung (12) eingebracht ist, **dadurch gekennzeichnet, dass** mindestens zwei linien- bzw. streifenförmig ausgebildete Einprägungen (12) vorgesehen sind, wobei jede Einprägung (12) zwei Eckpunkte des Stammblattes (2) verbindet, wobei die Einprägung (12) auf einer Seitenfläche des Stammblattes (2) eine Vertiefung und auf der gegenüberliegenden Seitenfläche eine korrespondierende Erhöhung (16) aufweist.

2. Sägeblatt nach Anspruch 1, **dadurch gekennzeichnet, dass** zumindest zwei diagonal verlaufende Einprägungen (12) in das Stammblatt (2) eingebracht sind, die diagonal liegende Eckpunkte verbinden.

3. Sägeblatt nach Anspruch 1 oder 2, **dadurch gekennzeichnet, dass** zumindest zwei in Längsrichtung und/oder Querrichtung verlaufende Einprägungen (12) in das Stammblatt (2) eingebracht sind, die jeweils zwei Eckpunkte verbinden.

4. Sägeblatt nach einem der Ansprüche 1 bis 3, **dadurch gekennzeichnet, dass** mindestens eine Aussparung (15) in das Stammblatt (2) eingebracht ist, die von mindestens einer Einprägung (12) begrenzt ist.

5. Sägeblatt nach Anspruch 4, **dadurch gekennzeichnet, dass** mehrere Aussparungen (15) in das Stammblatt (2) eingebracht sind, wobei mindestens zwei Aussparungen (15) von einer oder mehreren Einprägungen (12) separiert sind.

6. Sägeblatt nach Anspruch 4 oder 5, **dadurch gekennzeichnet, dass** die Aussparung (15) das Stammblatt (2) quer zu dessen Blattebene vollständig durchsetzt.

7. Sägeblatt nach einem der Ansprüche 1 bis 6, **dadurch gekennzeichnet, dass** die Aussparung (15) an allen Seiten von Einprägungen (12) begrenzt ist.

8. Sägeblatt nach einem der Ansprüche 1 bis 7, **dadurch gekennzeichnet, dass** die Einprägung (12) linien- bzw. streifenförmig ausgebildet ist.

9. Sägeblatt nach einem der Ansprüche 1 bis 8, **dadurch gekennzeichnet, dass** die mindestens eine Einprägung (12) durch Prägen in die Seitenfläche des Stammblatts (2) eingebracht ist.

10. Sägeblatt nach einem der Ansprüche 1 bis 9, **dadurch gekennzeichnet, dass** die Tiefe der Einprägung (12) mindestens 0.1 mm beträgt.

11. Sägeblatt nach einem der Ansprüche 1 bis 10, **dadurch gekennzeichnet, dass** die Breite der Einprägung (12) maximal ein Drittel der Blatthöhe beträgt.

12. Sägeblatt nach einem der Ansprüche 1 bis 11, **dadurch gekennzeichnet, dass** das Sägeblatt (1) lokale chemische Nachbearbeitungen, beispielsweise durch Beschichten aufweist.

13. Sägeblatt nach einem der Ansprüche 1 bis 12, **dadurch gekennzeichnet, dass** das Sägeblatt (1) lokale thermische Nachbearbeitungen, beispielsweise durch Induktionshärten aufweist.

14. Werkzeugmaschine, insbesondere Handwerkzeugmaschine mit einem Drehoszillationssägeblatt (1) nach einem der Ansprüche 1 bis 13.

## Claims

1. Rotary reciprocating saw blade for a power tool, in particular for a hand-operated power tool, comprising a blade body (2), which has an at least approximately rectilinear cutting edge (4), wherein at least one impression (12) is made in a side face of the blade body (2), **characterized in that** at least two linear or lamellar impressions (12) are provided, wherein each impression (12) connects two corner points of the blade body (2), wherein the impression (12) has a depression on one side face of the blade body (2) and has a corresponding elevation (16) on the opposite side face.

2. Saw blade according to Claim 1, **characterized in that** in the blade body (2) are made at least two diagonally running impressions (12), which connect diagonally situated corner points.

3. Saw blade according to Claim 1 or 2, **characterized in that** in the blade body (2) are made at least two longitudinally and/or transversely running impressions (12), which respectively connect two corner points.

4. Saw blade according to one of Claims 1 to 3, **characterized in that** in the blade body (2) is made at least one cavity (15), which is delimited by at least one impression (12).

5. Saw blade according to Claim 4, **characterized in that** in the blade body (2) are made a plurality of cavities (15), wherein at least two cavities (15) are separated by one or more impressions (12).

6. Saw blade according to Claim 4 or 5, **characterized in that** the cavity (15) passes fully through the blade body (2) transversely to the blade plane thereof.

7. Saw blade according to one of Claims 1 to 6, **characterized in that** the cavity (15) is delimited on all sides by impressions (12).

8. Saw blade according to one of Claims 1 to 7, **characterized in that** the impression (12) is of linear or lamellar configuration.

9. Saw blade according to one of Claims 1 to 8, **characterized in that** the at least one impression (12) is made by stamping in the side face of the blade body (2).

10. Saw blade according to one of Claims 1 to 9, **characterized in that** the depth of the impression (12) is at least 0.1 mm.

11. Saw blade according to one of Claims 1 to 10, **characterized in that** the width of the impression (12) is maximally one-third of the blade height.

12. Saw blade according to one of Claims 1 to 11, **characterized in that** the saw blade (1) has local chemical finishing treatments, for instance by coating.

13. Saw blade according to one of Claims 1 to 12, **characterized in that** the saw blade (1) has local thermal finishing treatments, for instance by induction hardening.

14. Power tool, in particular hand-operated power tool, comprising a rotary reciprocating saw blade (1) according to one of Claims 1 to 13.

## Revendications

1. Lame de scie oscillante rotative pour une machine-outil, en particulier pour une machine-outil portative, avec un corps de lame (2), qui présente une arête de coupe (4) au moins approximativement rectiligne, dans laquelle au moins une empreinte (12) est pratiquée dans une face latérale du corps de lame (2), **caractérisée en ce qu'**il est prévu au moins deux empreintes (12) réalisées en forme de lignes ou de bandes, dans laquelle chaque empreinte (12) relie deux coins du corps de lame (2), dans laquelle l'empreinte (12) sur une face latérale du corps de lame (2) présente un creux et sur la face latérale opposée une surélévation correspondante (16).

2. Lame de scie selon la revendication 1, **caractérisée en ce qu'**au moins deux empreintes (12) s'étendant en diagonale, qui relient des coins situés en diagonale, sont pratiquées dans le corps de lame (2).

3. Lame de scie selon la revendication 1 ou 2, **caractérisée en ce qu'**au moins deux empreintes (12) s'étendant en direction longitudinale et/ou en direction transversale, qui relient respectivement deux coins, sont pratiquées dans le corps de lame (2).

4. Lame de scie selon l'une quelconque des revendications 1 à 3, **caractérisée en ce qu'**au moins une découpe (15), qui est limitée par au moins une empreinte (12), est pratiquée dans le corps de lame (2).

5. Lame de scie selon la revendication 4, **caractérisée en ce que** plusieurs découpes (15) sont pratiquées dans le corps de lame (2), dans laquelle au moins deux découpes (15) sont séparées par une ou plusieurs empreinte(s) (12).

6. Lame de scie selon la revendication 4 ou 5, **caractérisée en ce que** la découpe (15) traverse entièrement le corps de lame (2) transversalement à son plan de lame.

7. Lame de scie selon l'une quelconque des revendications 1 à 6, **caractérisée en ce que** la découpe (15) est limitée de tous les côtés par des empreintes (12).

8. Lame de scie selon l'une quelconque des revendications 1 à 7, **caractérisée en ce que** l'empreinte (12) est réalisée en forme de ligne ou de bande.

9. Lame de scie selon l'une quelconque des revendications 1 à 8, **caractérisée en ce que** ladite au moins une empreinte (12) est pratiquée par gaufrage dans la face latérale du corps de scie (2).

10. Lame de scie selon l'une quelconque des revendications 1 à 9, **caractérisée en ce que** la profondeur de l'empreinte (12) vaut au moins 0,1 mm.

11. Lame de scie selon l'une quelconque des revendications 1 à 10, **caractérisée en ce que** la largeur de l'empreinte (12) vaut au maximum un tiers de la hauteur de la lame.

12. Lame de scie selon l'une quelconque des revendications 1 à 11, **caractérisée en ce que** la lame de scie (1) présente des parachèvements chimiques locaux, par exemple par revêtement.

13. Lame de scie selon l'une quelconque des revendications 1 à 12, **caractérisée en ce que** la lame de scie (1) présente des parachèvements thermiques locaux, par exemple par trempe par induction.

14. Machine-outil, en particulier machine-outil portative avec une lame de scie oscillante rotative (1) selon l'une quelconque des revendications 1 à 13.
